# EUROPÄISCHE PATENTANMELDUNG

(11) **EP 0 727 235 A2**
(43) Veröffentlichungstag der Anmeldung: **21.08.1996**
(21) Anmeldenummer: 96101151.7
(22) Anmeldetag: 27.01.1996
(51) Int. Cl.: A61M 11/02, B05B 1/00

(54) **Gerät zur Auftrennung des Teilchengrössenspektrums eines polydispersen Aerosols**

(30) Priorität: 17.02.1995 DE 19505341
(71) Anmelder: GSF - Forschungszentrum für Umwelt und Gesundheit GmbH, D-85764 Oberschleissheim (DE)
(72) Erfinder: Seeman, Stefan, D-82467 Garmisch-Patenkirchen (DE); Heyder, Joachim, Dr., D-80802 München (DE); Willeke, Klaus, Prof. Dr., Cincinatti, Ohio 45215 (DE)

(57) **Zusammenfassung**

Die Erfindung betrifft ein Gerät zur Auftrennung des Teilchengrößenspektrums eines polydispersen Aerosols bestehend aus einem Gehäuse mit einem Einlaßteil, das einen Aerosolkanal und Reingaszuführungskanal enthält und einem Trennteil, das mindestens zwei Abzugskanäle enthält, wobei sich zwischen Aerosolkanal und Abzugskanal eine Strömungsstrecke befindet, Aufgabe der Erfindung ist es, das Gerät so auszugestalten, daß aus einem polydispersen Aerosolstrom eine quasi-monodisperse Fraktion mit einem hohen Wirkungsgrad herausgefiltert werden kann.

Gelöst wird diese Aufgabe dadurch, daß der Aerosolkanal und der Reingaszuführungskanal derart ausgebildet ist, daß der Reingasstrom in den Aerosolstrom mündet und die Srömungsstrecke gerade ausgebildet ist, so daß der Reingas-und der Aerosolstrom ohne Umlenkung direkt auf die Einlassöffnung des Abzugskanals gerichtet ist.

## Beschreibung

Die Erfindung betrifft ein Gerät zur Auftrennung des Teilchengrößenspektrums eines polydispersen Aerosols nach dem Oberbegriff des Patentanspruchs 1.

Aerosole werden in der Medizin auf dem Gebiet der Inhalationstherapie eingesetzt. In der Umweltanalytik verbessern die e. g. Geräte die Analyse atmosphärischer Aerosole. Industrielle Aerosolprozesse können effektiver geführt werden.

Aus Prodi V., C. Melandri, T. DeZaiacomo, G. Tarroni, D. Hochrainer (1979). "An Inertial Spectrometer for Aerosol Particles". J. Aerosol Science, 10 (1979), S.411-419) ist ein gattungsgemäßes Gerät bekannt Inertial (Particle Aerosol Classifier (IPAC)). Mit dem IPAC besteht die Möglichkeit aus einem trockenen polydispersen Aerosolstrom einen quasi-monodispersen Anteil im Bereich von 0,8 - 8 µm, mit einer geometrischen Standardabweichung von 1,2 - 1,3 herauszufiltern.

Der IPAC besitzt zylindersymmetrische Geometrie und besteht aus zwei Teilen. In den oberen Teil (Trennteil) des Gerätes strömt mit Überdruck das polydisperse Aerosol zwischen zwei Schichten partikelfreier Mantelluft. Das Aerosol wird zusammen mit der Mantelluft durch eine Schlitzdüse auf eine 90°-Biegung hin beschleunigt. Beim Passieren der Biegung zwingt die Zentrifugalkraft die schwereren Teilchen sich auf einer Flugbahn mit größerem Radius zu bewegen als die der kleineren Teilchen. Der Gesamtfluß wird dann im unteren Teil (Klassifizierungsteil) durch drei konzentrisch angeordnete Eingangsschlitze in einen äußeren, mittleren und inneren Luftstrom aufgeteilt. Bei entsprechender Einstellung der verschiedenen Luftströme enthält der mittlere Strom eine Teilchenfraktion mit einer relativ engen Größenverteilung.

Des weiteren ist aus Conner, W.D. (1966). "An inertial-type particle seperator for collecting large samples". J. Air Pollut. Control Assoc. 16 (1966) S.35 ein virtueller Impaktor bekannt. Dieser setzt sich aus einer Beschleunigungsdüse und einer Sammelöffnung zusammen. Der Aerosolstrom wird durch die Düse zur Sammelöffnung hin beschleunigt, in der die Klassifizierung stattfindet. Ein kleiner Teil des Aerosolvolumenstroms wird durch die Sammelöffnung, der Hauptstrom in einem Winkel von 90° zur Düse abgesaugt. Entsprechend der unterschiedlichen Trägheit der verschieden großen Partikel gelangen diejenigen größer als die Trenngröße des Impaktors in die Sammelöffnung mit dem kleineren Volumenstrom. Der größere Volumenstrom enthält dann alle Partikel die kleiner als die Trenngröße des Impaktors sind. Die Partikel in beiden Luftströmen bleiben in luftgetragenem Zustand. Es existieren mehrere Abwandlungen dieses Prinzips um die Trennleistung des virtuellen Impaktors zu verbessern. Eine Methode ist das Opposing Jet Verfahren (Willeke K. and E. Pavlik "Size classification of fine particles by opposing jets". Environmental Science and Technology 12 (1978),S. 563-566), bei dem der virtuelle Impaktor nach dem Gegenstromprinzip arbeitet. Weitere Möglichkeiten bestehen darin, partikelfreies Reingas entweder dem kleineren Volumenstrom (Masuda H., D. Hochrainer, and W. Stöber "An improved virtual impactor for particle classification and generation of test aerosols with narrow size distributions". J. Aerosol Sci. 10 (1978), S. 275-287), oder dem polydispersen Aerosolstrom (Chen B. T. and H. C. Yeh "An improved virtual impactor: design and performance". J. Aerosol Sci. 18 (1987), S. 203-214) hinzuzufügen.

Ein Nachteil des IPAC liegt darin, daß er nur zur Klassifizierung eines trockenen Aerosols geeignet ist. Somit kann mit dem IPAC aus einem wässerigen polydispersen Aerosol, wie es z. B. ein Düsenvernebler liefert, keine quasi-monodisperse Fraktion herausgefiltert werden.

Die Ausbeute an quasi-monodispersen Aerosol im Vergleich zum polydispersen Ausgangsaerosol beträgt lediglich 10 %.

Der wesentliche Nachteil des virtuellen Impaktors liegt in seiner relativen unscharfen Trennleistung, da immer ein bestimmter Anteil der Partikel die kleiner als die Trenngröße des Impaktors sind, im Luftstrom der größeren Partikel enthalten ist. Mit dem virtuellen Impaktor findet somit nur eine starke Anreicherung und keine Auftrennung nach Größe der Partikel des polydispersen Aerosols statt. Auch kann der Größenbereich des klassifizierten Aerosols während des Betriebs des Impaktors nicht stufenlos verändert werden.

Aufgabe der Erfindung ist es, ein Gerät der e. g. Art so auszugestalten, daß aus einem polydispersen Aerosolstrom eine quasi-monodisperse Fraktion mit einem hohen Wirkungsgrad herausgefiltert werden kann.

Gelöst wird diese Aufgabe durch die kennzeichnenden Merkmale des Patentanspruchs 1. Die Unteransprüche beschreiben vorteilhafte Ausgestaltungen der Erfindug. Der Anspruch 6 nennt eine vorteilhafte Verwendung des Geräts.

Ein besonderer Vorteil des Geräts besteht darin, daß die Auftrennung des polydispersen Aerosols sowohl in 2 als auch 3 Volumenströme möglich ist.

Das Gerät hat noch eine Reihe weiterer Vorteile.

Es kann sowohl zur Einengung eines trockenen als auch wässerigen polydispersen Aerosols auf eine quasi-monodisperse Fraktion verwendet werden.

Die Materialkosten sind niedrig.

Die Bedienung ist einfach und unkompliziert.

Damit die Erfindung optimal arbeitet sind nur geringe Volumenströme notwendig, die leichter zu kontrollieren sind. Die quasi-monodisperse Fraktion weist eine geringe geometrische Standardabweichung auf.

Durch den Einsatz des Geräts in der Medizin zur Erzeugung monodisperser Medikamentenaerosole ist eine kontrollierte und gerichtete Deposition von Wirkstoffen im Atemtrakt möglich. Dies erlaubt eine Minimierung des Wirkstoffes, was zu einer starken Reduzierung schädlicher Nebenwirkungen führt.

Durch Änderung der einzelnen Volumenströme in den Kanälen des Trennteils kann der Größenbereich der quasi-monodispersen Fraktion sehr leicht gezielt verändert werden.

Die Erfindung wird im folgenden anhand eines Ausführungsbeispiels mit Hilfe der Figuren näher erläutert.

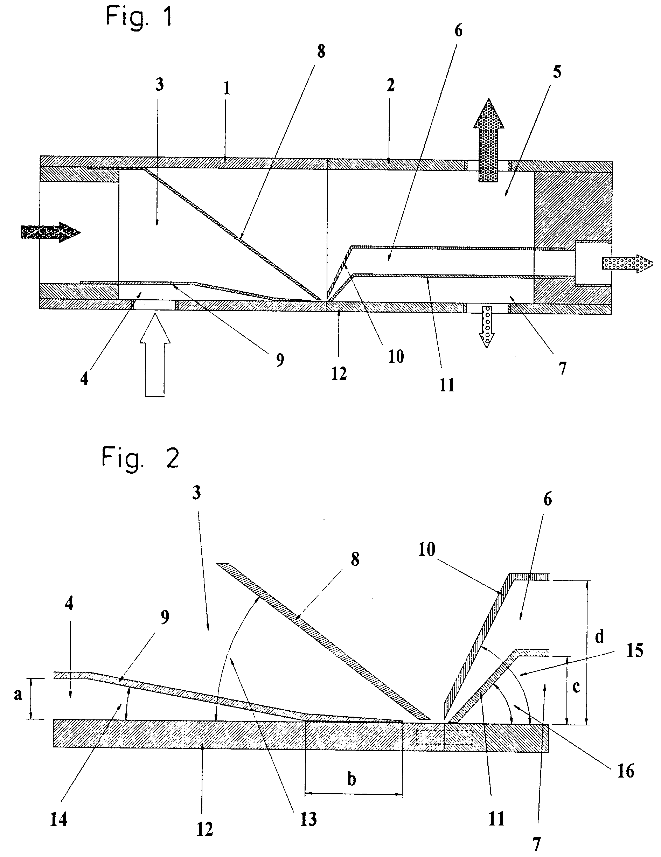

Dabei zeigt die Figur 1 den Aufbau des neu entwickelten Aerosol Size Classifiers (ASC) im Querschnitt.

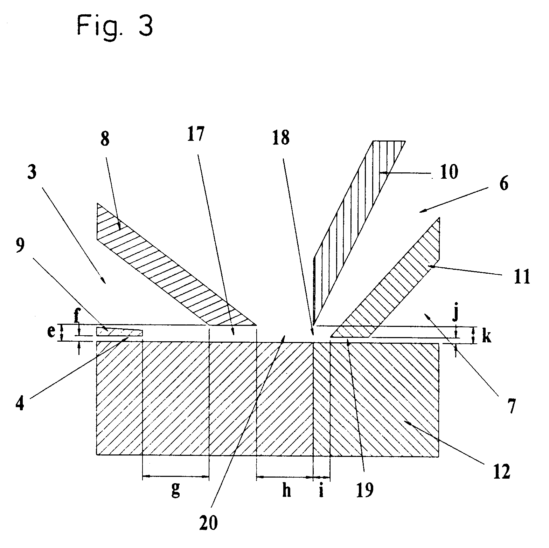

In Figur 2 und 3 ist der Mittelteil des ASC zur Verdeutlichung vergrößert dargestellt und mit Bemaßungen versehen. Die Abmessungen in Figur 2 und 3 beziehen sich auf eine mögliche Variante des Classifiers. Das Trennprinzip erlaubt je nach den Anforderungen verschiedene Abstände.

Der ASC, der in Fig. 1 dargestellt ist, besitzt einen rechtwinkeligen Querschnitt und besteht aus einem Gehäuse mit einem Einlaß- 1 und einem Trennteil 2. Einlaßteil 1 und Trennteil 2 können aus einem Teil bestehen oder aus zwei Teilen, die bündig und dicht aneinander gefügt sind.

Der Einlaßteil 1 enthält den Aerosolzuführungskanal 3 und den Reingaszuführungskanal 4, die jeweils links Einlaßöffnungen aufweisen (durch Pfeile gekennzeichnet) und die sich nach rechts hin zu ihren Auslassöffnungen hin verjüngen. Dabei endet der Reingaszuführungskanal 4 innerhalb des Aerosolzuführungskanals 3. Die beiden Kanäle 3, 4 werden von der Bodenplatte 12 und den beiden Begrenzungsplatten 8, 9 gebildet.

Der Auslaßöffnung des Aerosolzuführungskanals 3 schließen sich im Trennteil 2 drei Abzugskanäle 5, 6 und 7 für die verschiedenen Aerosolfraktionen an. Durch Pfeile sind Öffnungen in den Abzugskanälen 5, 6 und 7 gekennzeichnet. Diese Öffnungen dienen dem Abführen der einzelnen Aerosolfraktionen.Die Abzugskanäle 5, 6 und 7 werden durch die Bodenplatte 12, die Begrenzungsplatten 10, 11 und Teile des Gehäuses gebildet. Die Einlaßöffnungen der Abzugskanäle 6 und 7 liegen der Auslaßöffnung des Aerosolführungskanals gegenüber

In Fig. 2 ist ein Auschnitt des ASC mit Teilen der Bodenplatte 12 und Teilen der Begrenzungsplatten 8, 9, 10, und 11 welche die Auslassbereiche und Einlaßbereiche der Kanäle 4, 3, 6 und 7 bilden. Die Buchstaben a bis d bezeichnen Bemaßungen und die Bezugszeichen 13 bis 16 die Winkel der Platten 8 bis 11 gegen die Bodenplatte 12. Die Werte für Winkel und Abmessungen werden unten angegeben.

Fig. 3 zeigt den Ausschnitt um die Srömungsstrecke 20 mit den Auslaß- bzw. Einlaßöffnungen 17, 18 und 19 noch weiter vergrößert. Die Buchstaben e bis k bezeichnen Bemaßungen die ebenfalls weiter unten quantifiziert werden.

Aerosol- und partikelfreier Reingasstrom gelangen von links bzw. von unten (durch Pfeile dargestellt) in den Aerosol- und den Reingaszuführungskanal 3, 4 des Einlaßteils 1.

Der Winkel 13 zwischen Bodenplatte 12 und der Begrenzungsplatte 8 für den Aerosolzuführungskanal 3 soll zwischen 35° und 40° betragen, der Winkel 14 zwischen Bodenplatte 12 und Begrenzungsplatte 9 für den Reingaszuführungskanal 4 soll 15° nicht überschreiten. Die starke Verengung des Reingaszuführungskanals von a = 3 mm auf f = 0,1mm am Ende des Kanals führt zu einer 27 fach höheren Geschwindigkeit des Reingasstromes im Verhältnis zum Aerosolstrom. Dabei ist die Auslaßöffnung 17 mit e = 0,3 mm genau so groß wie die Einlassöffnung 18 mit k = 0.3mm. Der Reingaszuführungskanal hat dort wo er in den den Aerosolzuführungskanal mündet auf einer Länge b = 9 mm parallele Begrenzungen. Reingas- und Aerosolzuführungskanal sind so konstruiert, daß der Aerosolstrom erst 1,0 - 1,2 mm (g) vor der Auslaßöffnung 17 des Aerosolzuführungskanals mit dem Reingasstrom zusammengebracht wird. Dies verhindert eine Vermischung und Verwirbelung beider Gasströme vor der Auslaßöffnung 17. Reingas- und Aerosolstrom werden dann gemeinsam durch die Auslaßöffnung ohne Krümmung in den Trennteil beschleunigt.

Im sich anschließenden Trennteil wird der 1. Abzugskanal 7 durch die Bodenplatte 12 und der Begrenzungsplatte 11, der 2. Abzugskanal 6 durch die Begrenzungsplatte 11 und die Begrenzungsplatte 10 gebildet. Die Einlaßöffnung 19 des 1. Abzugskanals 7 weist eine Höhe j von 0,1 mm auf und ist im Verhältnis zur Einlaßöffnung 18 des 2. Abzugskanals 6 um i = 0,3 mm nach hinten versetzt. Die Einlaßöffnung 18 schließt in einem Abstand h von 1 mm direkt an die Auslaßöffnung 17 des Aerosolzuführungskanals 3 an und bildet dadurch eine freie Strömungsstrecke 20 aus. Die Auslaßöffnung 17 und die Einlaßöffnung 18 haben die gleiche Höhe von 0,3 mm. Der Abstand zwischen der Auslaßöffnung 17 und der Einlaßöffnung 18 und deren Höhen können in einem Bereich von 20 % von den oben genannten Werten abweichen.

Der Winkel 15 zwischen Bodenplatte 12 und der Begrenzungsplatte 10 kann zwischen 55° und 70°, der Winkel 16 zwischen Bodenplatte 12 und der Begrenzungsplatte 11 zwischen 40° und 50° variieren. Die Abzugskanäle 6 und 7 erweitern sich von ihren Einlassöffnungen aus bis aus eine Maximalweite. Die maximalen Abstände c und d der Platten 11 und 10 beträgt 5 und 10,6 mm.

Die Kanten der Begrenzungsplatten 8, 10 und 11 sind im Bereich der Öffnungen 17, 18 und 19 so ausgebildet wie in der Fig. 3 dargestellt. Geringe Abweichungen im Bereich von bis zu 10° sind zulässig.

Alle Winkel und Längenangaben können im Bereich von 10 % variiert werden.

Durch diese Anordnung der Abzugskanäle und verschieden starkes Absaugen wird der Gesamtsstrom aus Aerosol und Reingas nach der Auslaßöffnung des Aerosolzuführungskanals 17 in 3 Teilströme aufgeteilt. Entsprechend der Einstellungen der einzelnen Volumenströme in den Kanälen des Trennteils trennen sich die Partikel des polydispersen Aerosolstroms aufgrund ihrer unterschiedlichen Trägheit nach ihrer Größe auf. Im 1. Abzugskanal mit dem geringsten Volumenstrom finden sich wegen ihrer großen Trägheit die Partikel > 2,5 µm, im 2. Abzugskanal die Partikel 0,7 - 2,5 µm mit einer geometrischen Standardabweichung von 1,15 - 1,2 und im 3. Abzugskanal, mit dem höchsten Volumenstrom, die Partikel < 0,7 µm. 0,7 - 2,5 µm ist derjenige Größenbereich des quasi-monodispersen Aerosols, der mit dem vorliegendem Prototyp bei entsprechenden Einstellungen der Volumenströme erreicht werden kann. Durch geringfügige Veränderungen der Abstände läßt sich dieser Trennbereich weiter variieren.

Die Trennleistung des ASC bleibt zudem bestehen, wenn die Abzugskanäle im Trennteil von 3 auf 2 reduziert werden. Der ASC kann somit auch mit nur 2 Abzugskanälen betrieben werden, ohne daß sich dabei das Ergebnis verändert. Dabei entfällt der Abzugskanal 7. Das kann einfach durch Auswechslung des Trennteils 2 geschehen.

### Bezugszeichenliste

- 1: Einlaßteil
- 2: Trennteil
- 3: Aerosolzuführungskanal
- 4: Reingaszuführungskanal
- 5: 3. Abzugskanal
- 6: 2. Abzugskanal
- 7: 1. Abzugskanal
- 8: Begrenzungsplatte für Aerosolzuführungskanal
- 9: Begrenzungsplatte für Reingaszuführungskanal
- 10: Begrenzungsplatte für 3. Abzugskanal
- 11: Begrenzungsplatte für 2. Abzugskanal
- 12: Bodenplatte
- 13: Winkel der Begrenzungsplatte 8
- 14: Winkel der Begrenzungsplatte 9
- 15: Winkel der Begrenzungsplatte 10
- 16: Winkel der Begrenzungsplatte 11
- 17: Auslaßöffnung des Aerosolzuführungskanals
- 18: Einlaßöffnung des 2. Abzugskanal
- 19: Einlaßöffnung des 1. Abzugskanal
- 20: Strömungsstrecke

## Patentansprüche

1. Gerät zur Auftrennung des Teilchengrößenspektrums eines polydispersen Aerosols bestehend aus einem Gehäuse mit einem Einlaßteil, das einen Aerosolkanal und Reingaszuführungskanal enthält und einem Trennteil, das mindestens zwei Abzugskanäle enthält, wobei sich zwischen der Auslaßöffnung des Aerosolkanals und der Einlaßöffnung des einen Abzugskanals eine Strömungsstrecke befindet, dadurch gekennzeichnet, daß der Aerosolkanal (3) und der Reingaszuführungskanal (4) derart ausgebildet ist, daß der Reingasstrom in den Aerosolstrom mündet und die Srömungsstrecke (20) gerade ausgebildet ist, so daß der Reingas-und der Aerosolstrom ohne Umlenkung direkt auf die Einlaßöffnung des Abzugskanals (6) gerichtet ist.

2. Gerät nach Anspruch 1, dadurch gekennzeichnet, daß die lichte Weite der Einlaßöffnung (18) des Abzugskanals (6) genauso groß ist, wie die lichte Weite der Ausgangsöffnung (17) des Aerosolzuführungskanals (3).

3. Gerät nach Anspruch 1 oder 2, dadurch gekennzeichnet, daß ein dritter Abzugskanal (7) im Trennteil (2) eingebaut ist, welcher durch eine Begrenzungsplatte (11) von Abzugskanal 6 abgetrennt ist.

4. Gerät nach einem der Ansprüche 1 bis 3, dadurch gekennzeichnet, daß die Anordnung rotationssymmetrisch ist.

5. Gerät nach einem der Ansprüche 1 bis 4, dadurch gekennzeichnet, daß die Kanäle (3, 4, 5, 6, 7) rechteckige Querschnitte besitzen und daß Teile einer Begrenzungsfläche oder eine gesamte Begrenzungsfläche der Kanäle (3, 4, 5, 6, 7) durch eine Fläche (12) des Gehäuses gebildet werden.

6. Verwendung des Gerätes nach einem der Ansprüche 1 bis 5 zur Erzeugung von Aerosolen für die Therapie.
